(19) Europäisches Patentamt
European Patent Office
Office européen des brevets

(11) **EP 4 345 833 A1**

(12) **EUROPEAN PATENT APPLICATION**

(43) Date of publication:
**03.04.2024 Bulletin 2024/14**

(21) Application number: **22198712.6**

(22) Date of filing: **29.09.2022**

(51) International Patent Classification (IPC):
**G16H 30/20** (2018.01)    **G16H 50/20** (2018.01)
**G16H 50/70** (2018.01)

(52) Cooperative Patent Classification (CPC):
**G16H 30/20; G16H 30/40; G16H 50/20;
G16H 50/70**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Siemens Healthineers AG
91301 Forchheim (DE)**

(72) Inventors:
• **Fieselmann, Andreas
  91052 Erlangen (DE)**

• **Hümmer, Christian
  96215 Lichtenfels (DE)**
• **Biniazan, Ramyar
  90402 Nürnberg (DE)**

(74) Representative: **Siemens Healthineers
Patent Attorneys
Siemens Healthcare GmbH
SHS TE IP
Henkestraße 127
91052 Erlangen (DE)**

(54) **COMPUTER-IMPLEMENTED METHOD FOR A POST-ACQUISITION CHECK OF AN X-RAY IMAGE DATASET**

(57)     The invention relates to a computer-implemented method (10) for a post-acquisition check of an X-ray image dataset, comprising:
- Receiving input data (19), wherein the first input data is the X-ray image dataset comprising an X-ray image and first metadata,
- Applying (17) a trained function to the input data, wherein output data is generated, wherein the output data comprises second metadata, wherein the first metadata and the second metadata are compared,
- Providing (26) the output data, wherein the first metadata are confirmed in case the first metadata and the second metadata are in agreement, or the first metadata are suggested to be corrected with the second metadata in case the first metadata and the second metadata are not in agreement.

FIG 2

**Description**

The invention relates to a computer-implemented method for a post-acquisition check of an X-ray image dataset.

An X-ray imaging system, e.g. a radiography system or a fluoroscopy system, comprises an X-ray source and an X-ray detector. The examination object, in particular a patient, is arranged between the X-ray source and the X-ray detector so that an X-ray image of an examination region can be acquired.

In clinical routine, the human operator of a medical imaging device (e. g. X-ray system) must select the image acquisition protocol used for a specific organ to be acquired. It may happen that the operator accidentally or systematically selects an unsuitable protocol (e. g. a chest protocol for a hand to be acquired).

As an example, in an statistical analysis of a PACS dataset it has been found that the body part examined was incorrectly labeled in in 1% of cases and the selected clinical protocols (e.g., chest for hand images) has been incorrectly selected in 0.2% of cases. Wrongly chosen protocols lead to the following (clinical and technical) problems:

- Inadequately selected image acquisition protocols can lead to insufficient image quality, or more than the necessary dose applied to an organ.
- Inadequately selected image acquisition protocols can lead to incorrectly labeled images (e. g. incorrect body part examined) and limit the interoperability of those images in the clinical workflow or could even lead to wrong diagnosis.

In current clinical routine there is no dedicated check that the image acquisition protocol has been correctly selected. The operator of the imaging system or the radiologist interpreting the X-ray image may recognize by looking at the image and the meta information that the protocol was incorrectly selected.

It is an object of the invention to provide a computer-implemented method for a post-acquisition check of an X-ray image dataset, a computer-implemented method for providing a trained function, a checking system, a computer program product, a computer-readable medium, a training system, and an X-ray system, which allows a correction of the metadata before review of the X-ray image or at the time of the review of the X-ray image.

The object of the invention is solved by a computer-implemented method for a post-acquisition check of an X-ray image dataset according to claim 1, a computer-implemented method for providing a trained function according to claim 10, a checking system according to claim 11, a computer program product according to claim 12, a computer-readable medium according to claim 13, a training system according to claim 14, and an X-ray system according to claim 15.

In the following the solution according to the invention is described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the system.

Furthermore, in the following the solution according to the invention is described with respect to methods and systems for a post-acquisition check of an X-ray image dataset as well as with respect to methods and systems for the training of the trained function. Features, advantages or alternative embodiments herein can be assigned to the other claimed objects and vice versa. In other words, claims for methods and systems for training of the trained function can be improved with features described or claimed in context of the methods and systems for a post-acquisition check of an X-ray image dataset, and vice versa.

In particular, the trained function of the methods and systems for a post-acquisition check of an X-ray image dataset can be adapted by the methods and systems for training of the trained function. Furthermore, the input data can comprise advantageous features and embodiments of the training input data, and vice versa. Furthermore, the output data can comprise advantageous features and embodiments of the output training data, and vice versa.

The invention relates to a computer-implemented method for a post-acquisition check of an X-ray image dataset, comprising:

- Receiving input data, wherein the first input data is the X-ray image dataset comprising an X-ray image and first metadata,
- Applying a trained function to the input data, wherein output data is generated, wherein the output data comprises second metadata, wherein the first metadata and the second metadata are compared,
- Providing the output data, wherein the first metadata are confirmed in case the first metadata and the second metadata are in agreement, or the first metadata are suggested to be corrected with the second metadata in case the first metadata and the second metadata are not in agreement.

The X-ray image dataset comprises the X-ray image which means a 2D- or 3D-image with pixel or voxel values.

According to an aspect of the invention, the first metadata and the second metadata comprise information regarding the body part and/or the view position.

According to an aspect of the invention, the X-ray image dataset is a DICOM image dataset.

According to an aspect of the invention, the second metadata is automatically corrected.

According to an aspect of the invention, a suggestion is displayed to the user to correct the first metadata with the second metadata and the user can confirm or decline the suggestion.

According to an aspect of the invention, a private DICOM tag is added in case the first metadata is corrected with the second metadata.

According to an aspect of the invention, the trained function determines the body part and/or the view position of the examination region in the X-ray image.

According to an aspect of the invention, the second metadata is available when the X-ray image is reviewed.

According to an aspect of the invention, the trained function is based on a convolutional neural network.

The invention further relates to a computer-implemented method for providing a trained function, comprising:

- Receiving input training data, wherein the input training data comprises an X-ray image dataset comprising an X-ray image and first metadata,
- Receiving output training data, wherein the output training data is related to the input training data, wherein the output training data comprises second metadata,
- Training a function based on the input training data and the output training data,
- Providing the trained function.

During the training phase, the second metadata can correspond to the first metadata and/or the second metadata and the first metadata can be different. The second metadata can be determined by a specialist to create a groundtruth dataset which can be used as training data.

The invention further relates to a checking system, comprising:

- A first interface, configured for receiving first input data, wherein the first input data is an X-ray image dataset comprising an X-ray image and first metadata,
- A computation unit, configured for applying a trained function to the input data, wherein output data is generated, wherein the output data comprises second metadata, wherein the first metadata and the second metadata are compared,
- A second interface, configured for providing the output data, wherein the first metadata are confirmed in case the first metadata and the second metadata are in agreement, or the first metadata are suggested to be correct with the second metadata in case the first metadata and the second metadata are not in agreement.

The invention further relates to computer program product comprising instructions which, when the program is executed by a checking system, cause the checking system to carry out the method according to the invention.

The invention further relates to a computer-readable medium comprising instructions which, when executed by a providing system, cause the providing system to carry out the method according to the invention.

The invention further relates to a training system, comprising:

- A first training interface, configured for receiving input training data, wherein the input training data comprises an X-ray image dataset comprising an X-ray image and first metadata,
- A second training interface, configured for receiving output training data, wherein the output training data is related to the input training data, wherein the output training data comprises second metadata,
- A training computation unit, configured for training a function based on the input training data and the output training data,
- A third training interface, configured for providing the trained function.

The invention further relates to an X-ray system comprising the checking system according to the invention.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

In the following, abbreviations can be used:

- APAPC automatic post-acquisition protocol check
- BP body part examined
- BPVP body part & view position examined
- CNN convolutional neural network
- DICOM digital imaging and communications in medicine
- PACS picture archiving and communication system
- ML machine learning
- VP view position

A standard imaging workflow usually comprises the following steps, preferably in the following order:

1. Image exam is requested
2. Operator selects imaging protocol and positions patient and system
3. Operator acquires X-ray image dataset
4. X-ray image dataset is processed on system
5. Operator reviews acquired X-ray image dataset
6. X-ray image dataset gets send to database (e.g. PACS)

In this workflow several errors can occur. Table 1 shows possible scenarios of mismatches between requested exam, selected protocols and examined body part.

Scenario #1 is a correct workflow. Scenarios #2 and #4 will usually be recognized at the time of image interpretation. Scenario #3 is often not recognized by the operator when reviewing the acquired image or by the radiologist interpreting the image.

This invention aims to address especially scenario #3 such that this scenario is recognized automatically after the image has been acquired and necessary correction steps can be performed.

*Table 1: Examples of mismatches (scenarios #2, #3, #4) between requested exam and selected protocols and examined body part*

| scenario | requested exam of... | selected protocol for... | examined body part |
|---|---|---|---|
| #1 | Hand | Hand | Hand |
| #2 | Hand | Hand | Foot |
| #3 | Hand | Foot | Hand |
| #4 | Hand | Foot | Foot |

The selected image acquisition protocol determines the value for the body part (BP) and view position (VP) that the imaging system writes into the DICOM header. Thus, an incorrectly selected protocol may lead to an incorrect BP and/or VP in the DICOM header.

This problem is addressed by incorporating the proposed method, which can be called APAPC (automatic post-acquisition protocol check), into the imaging exam workflow. The APAPC estimates BP and VP from the image content and compares it to the respective information defined in the selected clinical protocol. Mismatches are incorporated as feedback into the review process to enable corrections of potentially wrong DICOM header entries for BP and VP. In the clinical workflow, this could be achieved by directly informing the operator or radiologist, e.g., with a pop-up window or suggestion for correct BP and VP. An alternative workflow is to automatically correct the DICOM header and facilitate retrospective quality control (e.g., by marking corrected cases with a specific private DICOM tag).

After the X-ray image (dataset) has been processed on the system it is automatically sent to the APAPC component. This APAPC component can be installed:

1. locally on the imaging system or
2. centrally at one location in the hospital or
3. outside of the hospital (cloud environment).

When the image is received by the APAPC it contains the pixel data and meta information. Usually, medical images are stored in DICOM format. In the DICOM format information about the body part examined can be stored using DICOM tags such as Anatomic Region Sequence (0008,2218) or Body Part Examined (0018,0015). Information about the view position (e. g. AP, lateral, ...) can be stored using DICOM tags such as View Position (0018,5101).

The main steps of the AI system using the trained function are described in the following subsections. The description is made generally for any type of medical imaging system but as a specific example an x-ray imaging system can be imagined.

In a first step, the trained function classifies the body part from pixel data. The trained function uses the image's pixel data to classify the image content. A trained machine learning model such as a convolutional neural network (CNN; such as DenseNet takes the pixel data as input and assigned probability scores to each class it is trained on.

A simple example of training a neural network to classify between different body regions is to provide a number of body parts to the neural network as classification. Prerequisite is a labeled dataset of (X-ray) images and respective annotations (e.g., body parts derived from the DICOM header and verified by manual inspection). Based on this, a classifier neural network (e.g., based on DenseNet architecture) maps the medical input image to a pre-defined number of relevant body parts. Depending on the content of the input image, the respective entries of the output vector are set to zero (body part not existing in the image) or equal to one (body part existing in the image). This implies that during training the neural network transforms a medical image (X-ray image) to a vector with one-hot encoding (all but one entry equal to zero). This concept is very general and can also be applied to different output vector definitions (e.g., tuples of body part and viewing position) or neural network architectures.

A body part can be examined using various view positions (AP, lateral, and more). To predict body parts acquired from different views, first BPVP (body part & view position) classes are created (e. g. "chest-AP" and "chest-lateral") on which the model is trained. Several BPVP classes can thus refer to one BP class.

The BPVP class with the highest probability score is denoted as $BPVP_{pixel}$; that is, the BP and VP combination predicted from the pixel data. From the $BPVP_{pixel}$ combined class one can determine the individual classes $BP_{pixel}$ and $VP_{pixel}$.

In a second step, the body part is extracted from meta information. The imaging system assigns BP and VP based on the selected protocol and stores this information in the DICOM header. To extract these classes (denoted as $BP_{meta}$ and $VP_{meta}$) one can query the value of the respective DICOM tags.

In a third step, a check for internal consistency is performed. The two BP classes ($BP_{pixel}$ and $BP_{meta}$) are compared, and the two VP classes ($VP_{pixel}$ and $VP_{meta}$) are compared. If they are equal, it is assumed that the correct protocol has been selected and no further action is required. If they any of them are unequal, then a review workflow is initiated (see next step).

In a fourth step, a review workflow takes place. When a mismatch is detected, the operator receives a message to review the acquired image and to determine if the class from the meta data (BP and/or VP) data should be replaced by the class predicted from the pixel data (BP and/or VP). Thus, the operator receives a recommended new BP and/or VP class.

At the same time, the operator should be made aware that an incorrectly selected acquisition protocol could lead to non-optimal image quality and the image should be carefully reviewed for image quality. Alternatively, the correction could be made automatically when the system has only a low number of false positive alerts.

The ML model or the trained function can be trained on data outside of the institution where it is being used or from retrospective data from the institution where is being used or a combination of both. A continuous learning approach can be used to improve the trained function further.

In addition, the ML model or trained function can be continuously improved by adding new images to a pool of training images. After the image has been reviewed by the operator this image can be sent to a central location for this institution where the pool of training images is stored. In regular intervals a new training of the ML model is triggered. The (re- )trained model can then be used in all upcoming prediction tasks.

If the prediction runs locally on the system, the model must be copied several times to each local system (e. g. by a Wifi connection). To run the trained function or prediction locally can be especially useful for mobile X-ray systems. If the prediction runs in a central location, then it only needs to be copied once.

The proposed method provides automatic checks of every X-ray image acquired for internal consistency (pixel data and meta data refer to same body part and view position). This is fundamentally different to a manual check.

The automated check reduces time and effort compared to a manual check and can lead to higher detection of inconsistencies (as the check may be forgotten by the human operator). The protocol check is made directly after image acquisition and before the image is sent to PACS. Thus, the images generated by the medical imaging system are of higher quality (less incorrect DICOM header information expected). This can be a competitive advantage of this particular imaging system compared to other systems. The advantage of the classification approach (predict a specific BPVP class) is that the operator is given a specific recommendation to which new BP and/or VP class the DICOM header entry should be changed. By the continuous learning approach, the system is capable to adapt to specific types of examinations that occur at the particular institution.

Examples of embodiments of the invention are explained in more detail below by means of drawings.

FIG 1 a schematic representation of the method for a post-acquisition check of an X-ray image dataset in a first embodiment;

FIG 2 a schematic representation of the method for a post-acquisition check of an X-ray image dataset in a second embodiment;

FIG 3 a schematic representation of a neural network according to the invention; and

FIG 4 a schematic representation of a convolutional neural network according to the invention; and

FIG 5 a schematic representation of an X-ray imaging system.

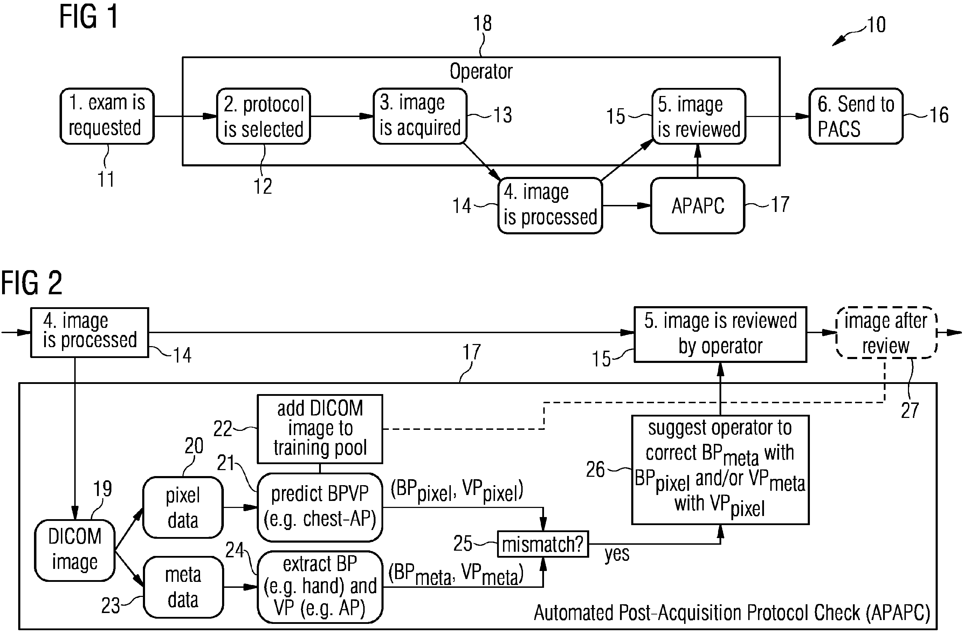

Fig. 1 displays a first embodiment of the method 10 for a post-acquisition check of an X-ray image dataset. In a first step 11, the exam is requested. In a second step 12, the protocol is selected by the operator. In a third step 13, the X-ray image is acquired. In a fourth step 14, the X-ray image is processed. In a fifth step 15, the X-ray image is reviewed. In a last step 16, the X-ray image is sent to PACS. After step 14 and before step 15, the trained function can be applied in step 17. The steps 12, 13, and 15 are performed in the operator domain 18.

Fig. 2 displays a second embodiment of the method for a post-acquisition check of an X-ray image dataset. After the image is processed in step 14, the DICOM image is received in step 19. Pixel data is extracted from the DICOM image in step 20. The trained function is applied to the pixel data in step 21 and a body part and view position are predicted. In step 23, the first meta data is extracted from the DICOM image. In step 24, the body part and the view position are extracted from the DICOM image. In step 25, the first metadata and the second metadata are compared. If there is a mismatch between the first metadata and the second metadata, second metadata as output are suggested and provided to the operator in step 26. Afterwards, the X-ray image is reviewed in step 15. In step 22, the DICOM image can be added to the training pool. In step 27, the X-ray image after review can be added to the training pool.

The computer-implemented method for a post-acquisition check of an X-ray image dataset, comprising the following steps:

- Receiving input data 19, wherein the first input data is the X-ray image dataset comprising an X-ray image and first metadata,
- Applying 17 a trained function to the input data, wherein output data is generated, wherein the output data comprises second metadata, wherein the first metadata and the second metadata are compared,
- Providing 26 the output data, wherein the first metadata are confirmed in case the first metadata and the second metadata are in agreement, or the first metadata are suggested to be corrected with the second metadata in case the first metadata and the second metadata are not in agreement.

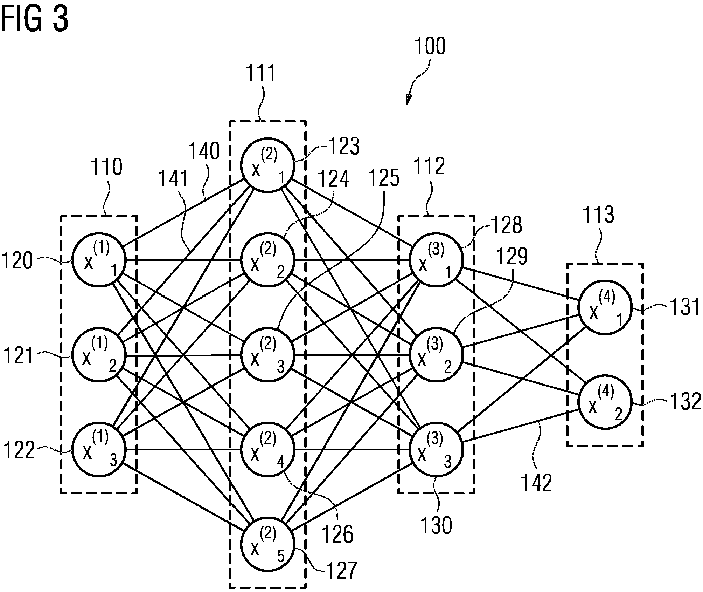

Fig. 3 displays an embodiment of an artificial neural network 100. Alternative terms for "artificial neural network" are "neural network", "artificial neural net" or "neural net".

The artificial neural network 100 comprises nodes 120, ..., 132 and edges 140, ..., 142, wherein each edge 140, ..., 142 is a directed connection from a first node 120, ..., 132 to a second node 120, ..., 132. In general, the first node 120, ..., 132 and the second node 120, ..., 132 are different nodes 120, ..., 132, it is also possible that the first node 120, ..., 132 and the second node 120, ..., 132 are identical. For example, in Fig. 1 the edge 140 is a directed connection from the node 120 to the node 123, and the edge 142 is a directed connection from the node 130 to the node 132. An edge 140, ..., 142 from a first node 120, ..., 132 to a second node 120, ..., 132 is also denoted as "ingoing edge" for the second node 120, ..., 132 and as "outgoing edge" for the first node 120, ..., 132.

In this embodiment, the nodes 120, ..., 132 of the artificial neural network 100 can be arranged in layers 110, ..., 113, wherein the layers can comprise an intrinsic order introduced by the edges 140, ..., 142 between the nodes 120, ..., 132. In particular, edges 140, ..., 142 can exist only between neighboring layers of nodes. In the displayed embodiment, there is an input layer 110 comprising only nodes 120, ..., 122 without an incoming edge, an output layer 113 comprising only nodes 131, 132 without outgoing edges, and hidden layers 111, 112 in-between the input layer 110 and the output layer 113. In general, the number of hidden layers 111, 112 can be chosen arbitrarily. The number of nodes 120, ..., 122 within the input layer 110 usually relates to the number of input values of the neural network, and the number of nodes 131, 132 within the output layer 113 usually relates to the number of output values of the neural network.

In particular, a (real) number can be assigned as a value to every node 120, ..., 132 of the neural network 100. Here, $x^{(n)}_i$ denotes the value of the i-th node 120, ..., 132 of the n-th layer 110, ..., 113. The values of the nodes 120, ..., 122 of the input layer 110 are equivalent to the input values of the neural network 100, the values of the nodes 131, 132 of the output layer 113 are equivalent to the output value of the neural network 100. Furthermore, each edge 140, ..., 142 can comprise a weight being a real number, in particular, the weight is a real number within the interval [-1, 1] or within the interval [0, 1]. Here, $w^{(m,n)}_{i,j}$ denotes the weight of the edge between the i-th node 120, ..., 132 of the m-th layer 110, ..., 113 and the j-th node 120, ..., 132 of the n-th layer 110, ..., 113. Furthermore, the abbreviation $w^{(n)}_{i,j}$ is defined for the weight $w^{(n,n+1)}_{i,j}$.

In particular, to calculate the output values of the neural network 100, the input values are propagated through the neural network. In particular, the values of the nodes 120, ..., 132 of the (n+1)-th layer 110, ..., 113 can be calculated based on the values of the nodes 120, ..., 132 of the n-th layer 110, ..., 113 by

$$x_j^{(n+1)} = f\left(\sum_i x_i^{(n)} \cdot w_{i,j}^{(n)}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values $w^{(m,n)}_{i,j}$ for the edges, the neural network 100 has to be trained using training data. In particular, training data comprises training input data and training output data (denoted as $t_i$). For a training step, the neural network 100 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 100 (backpropagation algorithm). In particular, the weights are changed according to $w'^{(n)}_{i,j} = w^{(n)}_{i,j} - \gamma \cdot \delta_j^{(n)} \cdot x_i^{(n)}$ wherein $\gamma$ is a learning rate, and the numbers $\delta^{(n)}_j$ can be recursively calculated as

$$\delta_j^{(n)} = \left(\sum_k \delta_k^{(n+1)} \cdot w_{j,k}^{(n+1)}\right) \cdot f'\left(\sum_i x_i^{(n)} \cdot w_{i,j}^{(n)}\right)$$

based on $\delta^{(n+1)}_j$, if the (n+1)-th layer is not the output layer, and

$$\delta_j^{(n)} = \left(x_k^{(n+1)} - t_j^{(n+1)}\right) \cdot f'\left(\sum_i x_i^{(n)} \cdot w_{i,j}^{(n)}\right)$$

if the (n+1)-th layer is the output layer 113, wherein f' is the first derivative of the activation function, and $y^{(n+1)}_j$ is the comparison training value for the j-th node of the output layer 113.

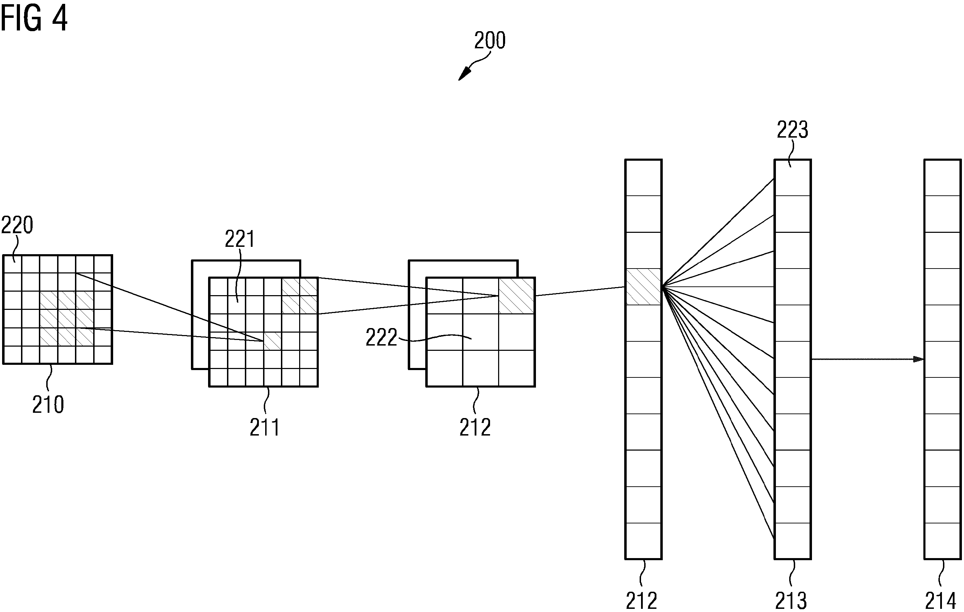

Fig. 4 displays an embodiment of a convolutional neural network 200. In the displayed embodiment, the convolutional neural network comprises 200 an input layer 210, a convolutional layer 211, a pooling layer 212, a fully connected layer 213 and an output layer 214. Alternatively, the convolutional neural network 200 can comprise several convolutional layers 211, several pooling layers 212 and several fully connected layers 213, as well as other types of layers. The order of the layers can be chosen arbitrarily, usually fully connected layers 213 are used as the last layers before the output layer 214.

In particular, within a convolutional neural network 200 the nodes 220, ..., 224 of one layer 210, ..., 214 can be considered to be arranged as a d-dimensional matrix or as a d-dimensional image. In particular, in the two-dimensional case the value of the node 220, ..., 224 indexed with i and j in the n-th layer 210, ..., 214 can be denoted as $x^{(n)}[i,j]$. However, the arrangement of the nodes 220, ..., 224 of one layer 210, ..., 214 does not have an effect on the calculations executed within the convolutional neural network 200 as such, since these are given solely by the structure and the weights of the edges.

In particular, a convolutional layer 211 is characterized by the structure and the weights of the incoming edges forming a convolution operation based on a certain number of kernels. In particular, the structure and the weights of the incoming edges are chosen such that the values $x^{(n)}_k$ of the nodes 221 of the convolutional layer 211 are calculated as

a convolution $x^{(n)}_k = K_k * x^{(n-1)}$ based on the values $x^{(n-1)}$ of the nodes 220 of the preceding layer 210, where the convolution * is defined in the two-dimensional case as

$$x_k^{(n)}[i,j] = (K_k * x^{(n-1)})[i,j] = \sum_{i'} \sum_{j'} K_k[i',j'] \cdot x^{(n-1)}[i-i', j-j'].$$

Here the k-th kernel $K_k$ is a d-dimensional matrix (in this embodiment a two-dimensional matrix), which is usually small compared to the number of nodes 220, ..., 224 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 220, ..., 224 in the respective layer 210, ..., 214. In particular, for a convolutional layer 211 the number of nodes 221 in the convolutional layer is equivalent to the number of nodes 220 in the preceding layer 210 multiplied with the number of kernels.

If the nodes 220 of the preceding layer 210 are arranged as a d-dimensional matrix, using a plurality of kernels can be interpreted as adding a further dimension (denoted as "depth" dimension), so that the nodes 221 of the convolutional layer 221 are arranged as a (d+1)-dimensional matrix. If the nodes 220 of the preceding layer 210 are already arranged as a (d+1)-dimensional matrix comprising a depth dimension, using a plurality of kernels can be interpreted as expanding along the depth dimension, so that the nodes 221 of the convolutional layer 221 are arranged also as a (d+1)-dimensional matrix, wherein the size of the (d+1)-dimensional matrix with respect to the depth dimension is by a factor of the number of kernels larger than in the preceding layer 210.

The advantage of using convolutional layers 211 is that spatially local correlation of the input data can exploited by enforcing a local connectivity pattern between nodes of adjacent layers, in particular by each node being connected to only a small region of the nodes of the preceding layer.

In the displayed embodiment, the input layer 210 comprises 36 nodes 220, arranged as a two-dimensional 6x6 matrix. The convolutional layer 211 comprises 72 nodes 221, arranged as two two-dimensional 6x6 matrices, each of the two matrices being the result of a convolution of the values of the input layer with a kernel. Equivalently, the nodes 221 of the convolutional layer 211 can be interpreted as arranges as a three-dimensional 6x6x2 matrix, wherein the last dimension is the depth dimension.

A pooling layer 212 can be characterized by the structure and the weights of the incoming edges and the activation function of its nodes 222 forming a pooling operation based on a nonlinear pooling function f. For example, in the two dimensional case the values $x^{(n)}$ of the nodes 222 of the pooling layer 212 can be calculated based on the values $x^{(n-1)}$ of the nodes 221 of the preceding layer 211 as

$$x^{(n)}[i,j] = f(x^{(n-1)}[id_1, jd_2], \dots, x^{(n-1)}[id_1+d_1-1, jd_2+d_2-1])$$

In other words, by using a pooling layer 212 the number of nodes 221, 222 can be reduced, by replacing a number $d_1 \cdot d_2$ of neighboring nodes 221 in the preceding layer 211 with a single node 222 being calculated as a function of the values of said number of neighboring nodes in the pooling layer. In particular, the pooling function f can be the max-function, the average or the L2-Norm. In particular, for a pooling layer 212 the weights of the incoming edges are fixed and are not modified by training.

The advantage of using a pooling layer 212 is that the number of nodes 221, 222 and the number of parameters is reduced. This leads to the amount of computation in the network being reduced and to a control of overfitting.

In the displayed embodiment, the pooling layer 212 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 213 can be characterized by the fact that a majority, in particular, all edges between nodes 222 of the previous layer 212 and the nodes 223 of the fully-connected layer 213 are present, and wherein the weight of each of the edges can be adjusted individually.

In this embodiment, the nodes 222 of the preceding layer 212 of the fully-connected layer 213 are displayed both as two-dimensional matrices, and additionally as non-related nodes (indicated as a line of nodes, wherein the number of nodes was reduced for a better presentability). In this embodiment, the number of nodes 223 in the fully connected layer 213 is equal to the number of nodes 222 in the preceding layer 212. Alternatively, the number of nodes 222, 223 can differ.

Furthermore, in this embodiment the values of the nodes 224 of the output layer 214 are determined by applying the Softmax function onto the values of the nodes 223 of the preceding layer 213. By applying the Softmax function, the sum of the values of all nodes 224 of the output layer is 1, and all values of all nodes 224 of the output layer are real numbers between 0 and 1. In particular, if using the convolutional neural network 200 for categorizing input data, the values of the output layer can be interpreted as the probability of the input data falling into one of the different categories.

A convolutional neural network 200 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = max(0,x)$, the tangent hyperbolics function or the sigmoid function.

In particular, convolutional neural networks 200 can be trained based on the backpropagation algorithm. For preventing overfitting, methods of regularization can be used, e.g. dropout of nodes 220, ..., 224, stochastic pooling, use of artificial data, weight decay based on the L1 or the L2 norm, or max norm constraints.

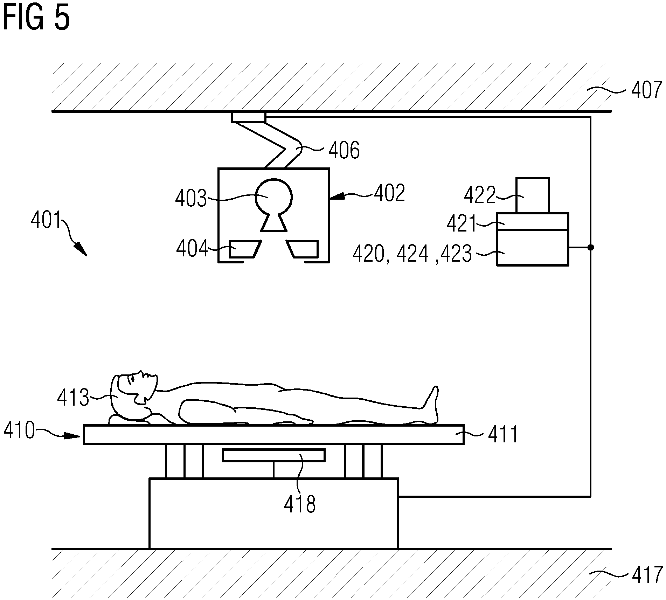

Fig. 5 shows an exemplary embodiment of an X-ray (imaging) system 401, especially a radiography system, according to the invention. The X-ray system 401 has a patient positioning device 410 with a table 411 fixed to the floor 417. The object 413 lies on the table 411. The patient positioning device 410 further comprises an X-ray detector unit 418.

The X-ray system 401 comprises an X-ray source 403 and an X-ray detector unit 418. The X-ray source unit 402, which comprises the X-ray source 403 and a collimator unit 404. The X-ray source unit 402 can be connected to the ceiling 407 of the examination room by means of a ceiling mount 406. By means of the ceiling mount 406, the X-ray source 403 can be moved.

The X-ray system 401 may also comprise an input unit 421 and an output unit 422. The input unit 421 and the output unit 422 may be connected to the control unit 420. The control unit 420 may comprise the checking system. The control unit 420 may further comprise or be connected to the training system 424.

Although the invention has been further illustrated in detail by the preferred embodiments, the invention is not limited by the disclosed examples and other variations may be derived therefrom by those skilled in the art without departing from the scope of protection of the invention.

**Claims**

1. A computer-implemented method (10) for a post-acquisition check of an X-ray image dataset, comprising:

   - Receiving input data (19), wherein the first input data is the X-ray image dataset comprising an X-ray image and first metadata,
   - Applying (17) a trained function to the input data, wherein output data is generated, wherein the output data comprises second metadata, wherein the first metadata and the second metadata are compared,
   - Providing (26) the output data, wherein the first metadata are confirmed in case the first metadata and the second metadata are in agreement, or the first metadata are suggested to be corrected with the second metadata in case the first metadata and the second metadata are not in agreement.

2. A method according to claim 1, wherein the first metadata and the second metadata comprise information regarding the body part and/or the view position.

3. A method according to one of the preceding claims, wherein the X-ray image dataset is a DICOM image dataset.

4. A method according to one of the preceding claims, wherein the second metadata is automatically corrected.

5. A method according to one of the preceding claims, wherein the suggestion to correct the first metadata with the second metadata is displayed to the user and the user can confirm or decline the suggestion.

6. A method according to one of the preceding claims, wherein a private DICOM tag is added in case the first metadata is corrected by using the second metadata.

7. A method according to one of the preceding claims, wherein the trained function determines the body part and/or the view position of the examination region in the X-ray image.

8. A method according to one of the preceding claims, wherein the second metadata is available when the X-ray image is reviewed.

9. A method according to one of the preceding claims, wherein the trained function is based on a convolutional neural network.

10. A computer-implemented method for providing a trained function, comprising:

- Receiving input training data, wherein the input training data comprises an X-ray image dataset comprising an X-ray image and first metadata,
- Receiving output training data, wherein the output training data is related to the input training data, wherein the output training data comprises second metadata,
- Training a function based on the input training data and the output training data,
- Providing the trained function.

11. A checking system (423), comprising:

- A first interface, configured for receiving first input data, wherein the first input data is an X-ray image dataset comprising an X-ray image and first metadata,
- A computation unit, configured for applying a trained function to the input data, wherein output data is generated, wherein the output data comprises second metadata, wherein the first metadata and the second metadata are compared,
- A second interface, configured for providing the output data, wherein the first metadata are confirmed in case the first metadata and the second metadata are in agreement, or the first metadata are suggested to be corrected with the second metadata in case the first metadata and the second metadata are not in agreement.

12. A computer program product comprising instructions which, when the program is executed by a checking system, cause the checking system to carry out the method of one of the claims 1 to 9.

13. A computer-readable medium comprising instructions which, when executed by a providing system, cause the providing system to carry out the method of one of the claims 1 to 9.

14. A training system (424), comprising:

- A first training interface, configured for receiving input training data, wherein the input training data comprises an X-ray image dataset comprising an X-ray image and first metadata,
- A second training interface, configured for receiving output training data, wherein the output training data is related to the input training data, wherein the output training data comprises second metadata,
- A training computation unit, configured for training a function based on the input training data and the output training data,
- A third training interface, configured for providing the trained function.

15. An X-ray system (401) comprising the checking system according to claim 11.

FIG 1

FIG 2

FIG 3

EP 4 345 833 A1

FIG 4

FIG 5

Europäisches
Patentamt

European
Patent Office

Office européen
des brevets

# EUROPEAN SEARCH REPORT

Application Number

**EP 22 19 8712**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/211678 A1 (FOLEY CHRISTOPHER [GB] ET AL) 2 July 2020 (2020-07-02)<br>* paragraph [0013] *<br>* paragraph [0030] – paragraph [0035] *<br>* paragraph [0049] *<br>* paragraph [0064] – paragraph [0067] *<br>* paragraph [0071] – paragraph [0072] *<br>* paragraph [0090] *<br>* paragraph [0088] *<br>* paragraph [0094] – paragraph [0095] *<br>* paragraph [0101] – paragraph [0105] *<br>* figures * | 1-15 | INV.<br>G16H30/20<br>G16H50/20<br>G16H50/70 |
| A | EP 3 872 757 A1 (GE PREC HEALTHCARE LLC [US]) 1 September 2021 (2021-09-01)<br>* paragraph [0016] – paragraph [0018] * | 1-15 | |
| A | US 2019/164285 A1 (NYE KATELYN ROSE [US] ET AL) 30 May 2019 (2019-05-30)<br>* paragraph [0125] – paragraph [0126] *<br>* paragraph [0160] – paragraph [0162] * | 1-15 | |
| | | | TECHNICAL FIELDS SEARCHED (IPC)<br><br>G16H |

The present search report has been drawn up for all claims

| Place of search | Date of completion of the search | Examiner |
|---|---|---|
| Munich | 22 February 2023 | Megalou-Nash, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 8712

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2023

| Patent document cited in search report | | Publication date | Patent family member(s) | | Publication date |
|---|---|---|---|---|---|
| US 2020211678 | A1 | 02-07-2020 | EP | 3903320 A1 | 03-11-2021 |
| | | | US | 2020211678 A1 | 02-07-2020 |
| | | | WO | 2020136119 A1 | 02-07-2020 |
| EP 3872757 | A1 | 01-09-2021 | CN | 113393417 A | 14-09-2021 |
| | | | EP | 3872757 A1 | 01-09-2021 |
| | | | US | 2021271931 A1 | 02-09-2021 |
| | | | US | 2021350186 A1 | 11-11-2021 |
| US 2019164285 | A1 | 30-05-2019 | US | 2019164285 A1 | 30-05-2019 |
| | | | US | 2020372651 A1 | 26-11-2020 |

EPO FORM P0459

For more details about this annex : see Official Journal of the European Patent Office, No. 12/82